# EUROPEAN PATENT APPLICATION

(11) **EP 3 680 860 A1**
(43) Date of publication of application: **15.07.2020**
(21) Application number: 20150964.3
(22) Date of filing: 09.01.2020
(51) Int. Cl.: G06T 11/00

(54) **TOMOGRAPHIC IMAGING APPARATUS AND METHOD OF GENERATING TOMOGRAPHIC IMAGE**

(30) Priority: 10.01.2019 KR 20190003285
(71) Applicant: Samsung Electronics Co., Ltd., Gyeonggi-do 16677 (KR)
(72) Inventor: Yoon, Huisu, 16677 Suwon-si, Gyeonggi-do (KR); Lee, Kyoung-Yong, 16677 Suwon-si, Gyeonggi-do (KR); Jo, Jaemoon, 16677 Suwon-si, Gyeonggi-do (KR)
(74) Representative: Gulde & Partner

(57) **Abstract**

A method of generating a tomographic image includes radiating transmission X-rays onto an object and acquiring X-ray data by detecting reflected X-rays reflected from the object; generating a first tomographic image by reconstructing the X-ray data; identifying, in the first tomographic image, a first region corresponding to a metal particle included in the object; generating a second tomographic image by changing a pixel value of the first region to a predetermined value; acquiring first projection data by performing forward projection on the second tomographic image; generating second projection data by performing interpolation on first signal intensity values corresponding to the first region, based on the first projection data; generating reconstruction data by subtracting the first projection data from the second projection data; generating a third tomographic image based on the reconstruction data; and generating a fourth tomographic image based on the third tomographic image and the first tomographic image.

## Description

### CROSS-REFERENCE TO RELATED APPLICATION

This application is based on and claims priority under 35 U.S.C. §119 to Korean Patent Application No. 10-2019-0003285, filed on January 10, 2019, in the Korean Intellectual Property Office, the disclosure of which is incorporated by reference herein in its entirety.

### BACKGROUND

### 1. Field

The disclosure relates to a tomographic imaging apparatus and a method, performed by the tomographic imaging apparatus, of generating a tomographic image.

### 2. Description of Related Art

Medical imaging apparatuses are used to obtain images showing an internal structure of an object. The medical imaging apparatuses are non-invasive examination apparatuses that capture and process images of details of structures, tissue, fluid flow, etc., inside a body and display the images to a user. The user, e.g., a medical practitioner, may use medical images output from the medical imaging apparatuses to diagnose a patient's condition and diseases.

A computed tomography (CT) apparatus is a representative example of an apparatus for imaging an object by emitting X-rays toward a patient.

The CT apparatus that is a tomographic imaging apparatus among medical imaging apparatuses is capable of providing a cross-sectional image of an object and may represent an internal structure (e.g., organs such as a kidney, a lung, etc.) of the object without superimposition of adjacent structures, as compared to a general X-ray apparatus. Due to these advantages, CT apparatuses have been widely used for precise diagnosis of diseases. Hereinafter, a medical image obtained by a tomographic imaging apparatus is referred to as a tomographic image.

To obtain a tomographic image, tomographic imaging is performed on an object by using a tomographic imaging apparatus to acquire raw data. The acquired raw data is then used to reconstruct a tomographic image. In this case, the raw data may be projection data acquired by projecting X-rays onto the object, or may be a sinogram representing a set of projection data.

However, metal artifacts, caused by metals in the object, may be present in a tomographic image generated by a tomographic imaging apparatus of the related art. The metal artifacts occurring in the tomographic image may degrade accuracy in image analysis and disease diagnosis by the user such as a medical doctor.

Thus, there is a need for methods of generating a tomographic image from which a metal artifact is removed.

### SUMMARY

Provided are a tomographic imaging apparatus for generating a tomographic image from which a metal artifact caused by a metal in an object is removed, and a method, performed by the tomographic imaging apparatus, of generating a tomographic image.

Additional aspects will be set forth in part in the description which follows and, in part, will be apparent from the description, or may be learned by practice of the presented embodiments.

In accordance with an aspect of the disclosure, a method of generating a tomographic image is performed by a tomographic imaging apparatus and includes radiating transmission X-rays onto an object and acquiring X-ray data by detecting reflected X-rays reflected from the object; generating a first tomographic image by reconstructing the X-ray data; identifying, in the first tomographic image, a first region corresponding to a metal particle included in the object; generating a second tomographic image by changing a pixel value of the first region to a predetermined value; acquiring first projection data by performing forward projection on the second tomographic image; generating second projection data by performing interpolation on first signal intensity values corresponding to the first region, based on the first projection data; generating reconstruction data by subtracting the first projection data from the second projection data; generating a third tomographic image based on the reconstruction data, wherein the third tomographic image includes correction data corresponding to a metal artifact caused by the metal particle; and generating a fourth tomographic image based on the third tomographic image and the first tomographic image.

The generating of the second tomographic image may include setting the predetermined value based on attribute data of the metal artifact in the first tomographic image.

The generating of the second tomographic image may include setting the predetermined value based on a pixel value of a second region located near the first region.

The generating of the second tomographic image may include setting the predetermined value based on a part of the object corresponding to the first tomographic image.

The acquiring of the first projection data may include performing the forward projection on the second tomographic image by projecting a virtual parallel beam onto the second tomographic image.

The acquiring of the first projection data may include: setting, based on a position of the metal particle, at least one projection angle of the forward projection; and acquiring the first projection data by performing the forward projection on the second tomographic image at the at least one projection angle.

The acquiring of the first projection data may include: setting, based on a position of the metal particle, an interval of projection angles of the forward projection; and acquiring the first projection data by performing the forward projection on the second tomographic image at the interval.

The generating of the second projection data may include: comparing the first signal intensity values in the first projection data with second signal intensity values obtained by performing linear interpolation on the first signal intensity values; selecting, based on attribute data of the metal artifact in the first tomographic image, one from among third signal intensity values greater than the second signal intensity values from among the first signal intensity values, or fourth signal intensity values less than the second signal intensity values from among the first signal intensity values; obtaining fifth signal intensity values by calculating a difference between the second signal intensity values and the selected one from among the third signal intensity values or the fourth signal intensity values; and generating the second projection data based on the obtained fifth signal intensity value.

The attribute data of the metal artifact may include a pixel value of a third region corresponding to the metal artifact, and the selecting of the one from among the third signal intensity values or the fourth signal intensity values may include: selecting the third signal intensity values based on a comparison result indicating that the pixel value of the third region is greater than a pixel value of a fourth region located near the third region; and selecting the fourth signal intensity values based on a comparison result indicating that the pixel value of the third region is less than the pixel value of the fourth region.

A computer program product may include a non-transitory computer-readable storage medium having recorded thereon instructions for executing the methods described herein on a computer.

In accordance with an aspect of the disclosure, a tomographic imaging apparatus includes an X-ray radiator configured to radiate transmission X-rays onto an object; an X-ray detector configured to acquire X-ray data by detecting reflected X-rays reflected from the object; and at least one processor configured to: generate a first tomographic image by reconstructing the X-ray data; identify, in the first tomographic image, a first region corresponding to a metal particle included in the object; generate a second tomographic image by changing a pixel value of the first region to a predetermined value; acquire first projection data by performing forward projection on the second tomographic image; generate second projection data by performing interpolation on first signal intensity values corresponding to the first region based on the first projection data; generate reconstruction data by subtracting the first projection data from the second projection data; generate a third tomographic image based on the reconstruction data, wherein the third tomographic image includes correction data corresponding to a metal artifact caused by the metal particle; and generate a fourth tomographic image based on the third tomographic image and the first tomographic image.

The at least one processor may be further configured to set the predetermined value based on attribute data of the metal artifact in the first tomographic image.

The at least one processor may be further configured to set the predetermined value based on a pixel value of a second region located near the first region.

The at least one processor may be further configured to set the predetermined value based on a part of the object corresponding to the first tomographic image.

The at least one processor may be further configured to perform the forward projection on the second tomographic image by projecting a virtual parallel beam onto the second tomographic image.

The at least one processor may be further configured to: set, based on a position of the metal particle, at least one projection angle of the forward projection; and acquire the first projection data by performing the forward projection on the second tomographic image at the at least one projection angle.

The at least one processor may be further configured to: set, based on a position of the metal particle, an interval of projection angles of the forward projection; and acquire the first projection data by performing the forward projection on the second tomographic image at the interval.

The at least one processor may be further configured to: compare the first signal intensity values in the first projection data with second signal intensity values obtained by performing linear interpolation on the first signal intensity values; select, based on attribute data of the metal artifact in the first tomographic image, one from among third signal intensity values greater than the second signal intensity values from among the first signal intensity values, or fourth signal intensity values less than the second signal intensity values from among the first signal intensity values; obtain fifth signal intensity values by calculating a difference between the second signal intensity values and the selected one from among the third signal intensity values or the fourth signal intensity values; and generate the second projection data based on the obtained fifth signal intensity value.

The attribute data of the metal artifact may include a pixel value of a third region corresponding to the metal artifact, and the at least one processor may be further configured to: select the third signal intensity values based on a comparison result indicating that the pixel value of the third region is greater than a pixel value of a fourth region located near the third region; and select the fourth signal intensity values based on a comparison result indicating that the pixel value of the third region is less than the pixel value of the fourth region.

### BRIEF DESCRIPTION OF THE DRAWINGS

The above and other aspects, features, and advantages of certain embodiments will be more apparent from the following description taken in conjunction with the accompanying drawings, in which:
FIG. 1 illustrates a structure of a tomographic imaging system according to an embodiment;
FIG. 2 is a flowchart of a method of generating a tomographic image, according to an embodiment;
FIG. 3 illustrates an example of changing a pixel value of a region corresponding to a metal particle in a tomographic image, according to an embodiment;
FIG. 4 illustrates acquisition of projection data by performing forward projection on a tomographic image, according to an embodiment;
FIG. 5 illustrates setting of a projection angle at which forward projection is to be performed, according to an embodiment;
FIG. 6 illustrates setting of an interval of projection angles at which forward projection is to be performed, according to an embodiment;
FIG. 7 illustrates a subtraction image obtained by performing interpolation and then filtering on a signal intensity value in projection data, according to an embodiment;
FIG. 8 illustrates generation of a tomographic image including correction data for a metal artifact, according to an embodiment;
FIG. 9 illustrates a comparison between projection data from which a signal intensity value corresponding to a metal particle is removed and projection data that has undergone interpolation, according to an embodiment;
FIG. 10 illustrates projection data generated for removing a white streak metal artifact, according to an embodiment;
FIG. 11 illustrates projection data generated for removing a white streak metal artifact, according to an embodiment;
FIG. 12 illustrates a tomographic image including correction data for removing a white streak metal artifact, according to an embodiment;
FIG. 13 illustrates projection data generated for removing a black shading metal artifact, according to an embodiment;
FIG. 14 illustrates projection data generated for removing a black shading metal artifact, according to an embodiment;
FIG. 15 illustrates a tomographic image including a correction value for removing a black shading metal artifact, according to an embodiment;
FIG. 16 illustrates a tomographic image from which a metal artifact is removed, according to an embodiment; and
FIG. 17 is a block diagram of a structure of a tomographic imaging apparatus according to an embodiment.

### DETAILED DESCRIPTION

Embodiments are disclosed so that the scope of the present disclosure is clarified and one of ordinary skill in the art to which the present disclosure pertains may implement embodiments of the present disclosure. The disclosed embodiments may have various forms.

Throughout the specification, like reference numerals or characters refer to like elements. In the present specification, all elements of embodiments are not explained, but general matters in the technical field of the present disclosure or redundant matters between embodiments will not be described. Terms "module" or "unit" used herein may be implemented using at least one or a combination from among software, hardware, or firmware, and, according to embodiments, a plurality of "module" or "unit" may be implemented using a single element, or a single "module" or "unit" may be implemented using a plurality of units or elements. Examples of operational principles of the present disclosure and embodiments thereof will now be described more fully with reference to the accompanying drawings.

In the present specification, an image may include a medical image obtained by a medical imaging apparatus, such as a computed tomography (CT) apparatus, a magnetic resonance imaging (MRI) apparatus, an ultrasound imaging apparatus, or an X-ray apparatus.

Throughout the specification, the term "object" is a thing to be imaged, and may include a human, an animal, or a part of a human or animal. For example, the object may include a part of a body (i.e., an organ), a phantom, or the like.

In the present specification, a "tomographic imaging system" or "tomographic imaging apparatus" refers to a system or apparatus configured to emit X-rays while rotating around at least one axis relative to an object and photograph the object by detecting the X-rays.

In the specification, a "CT image" refers to an image constructed from raw data obtained by photographing an object by detecting X-rays that are emitted as the tomographic imaging system or apparatus rotates about at least one axis with respect to the object.

It will be understood that, although the terms including an ordinal number such as "first", "second", etc. may be used herein to describe various elements or components, these elements or components should not be limited by the terms. The terms are only used to distinguish one element or component from another element or component.

Throughout the disclosure, the expression "at least one of a, b or c" indicates only a, only b, only c, both a and b, both a and c, both b and c, all of a, b, and c, or variations thereof.

Hereinafter, embodiments will be described in detail with reference to the accompanying drawings.

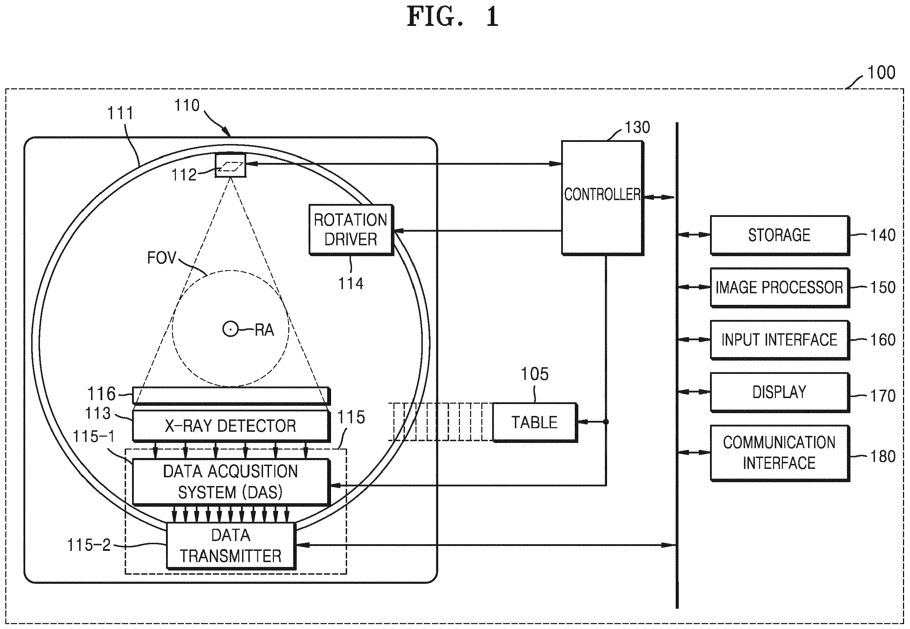

FIG. 1 illustrates a structure of a tomographic imaging system 100 according to an embodiment.

The tomographic imaging system 100 may include a gantry 110, a table 105, a controller 130, a storage 140, an image processor 150, an input interface 160, a display 170, and a communication interface 180.

The gantry 110 may include a rotating frame 111, an X-ray generator 112, an X-ray detector 113, a rotation driver 114, and a readout device 115.

The rotating frame 111 may receive a driving signal from the rotation driver 114 and rotate around a rotation axis (RA).

An anti-scatter grid 116 may be disposed between an object and the X-ray detector 113 and may transmit most of primary radiation and attenuate scattered radiation. The object may be positioned on the table 105 which may move, tilt, or rotate during a CT scan.

The X-ray generator 112 receives a voltage and a current from a high voltage generator (HVG) to generate and emit X-rays.

The tomographic imaging system 100 may be implemented as a single-source tomographic imaging system including one X-ray generator 112 and one X-ray detector 113, or as a dual-source tomographic imaging system including two X-ray generators 112 and two X-ray detectors 113.

The X-ray detector 113 detects radiation that has passed through the object. For example, the X-ray detector 113 may detect radiation by using a scintillator, a photon counting detector, etc.

Methods of driving the X-ray generator 112 and the X-ray detector 113 may vary depending on scan modes used for scanning of the object. The scan modes are classified into an axial scan mode and a helical scan mode, according to a path along which the X-ray detector 113 moves. Furthermore, the scan modes are classified into a prospective mode and a retrospective mode, according to a time interval during which X-rays are emitted.

The controller 130 may control an operation of each of the components of the tomographic imaging system 100. The controller 130 may include a memory configured to store program for performing a function or data and a processor configured to process the program codes or the data. The controller 130 may be implemented in various combinations of at least one memory and at least one processor. The processor may generate or delete a program module according to an operating status of the tomographic imaging system 100 and process operations of the program module.

The readout device 115 receives a detection signal generated by the X-ray detector 113 and outputs the detection signal to the image processor 150. The readout device 115 may include a data acquisition system (DAS) 115-1 and a data transmitter 115-2. The DAS 115-1 uses at least one amplifying circuit to amplify a signal output from the X-ray detector 113, and outputs the amplified signal. The data transmitter 115-2 uses a circuit such as a multiplexer (MUX) to output the signal amplified in the DAS 115-1 to the image processor 150. According to a slice thickness or a number of slices, only some of a plurality of pieces of data collected by the X-ray detector 113 may be provided to the image processor 150, or the image processor 150 may select only some of the plurality of pieces of data.

The image processor 150 obtains tomographic data from a signal obtained by the readout device 115 (e.g., pure data that is data before being processed). The image processor 150 may pre-process the obtained signal, convert the obtained signal into tomographic data, and post-process the tomographic data. The image processor 150 may perform some or all of the processes described herein, and the type or order of processes performed by the image processor 150 may vary according to embodiments.

The image processor 150 may perform pre-processing, such as a process of correcting sensitivity irregularity between channels, a process of correcting a rapid decrease of signal strength, or a process of correcting signal loss due to an X-ray absorbing material, on the signal obtained by the readout device 115.

According to embodiments, the image processor 150 may perform some or all of the processes for reconstructing a tomographic image, to thereby generate the tomographic data. According to an embodiment, the tomographic data may be in the form of data that has undergone back-projection, or in the form of a tomographic image. According to embodiments, additional processing may be performed on the tomographic data by an external device such as a server, a medical apparatus, or a portable device.

Raw data is a set of data values corresponding to intensities of X-rays that have passed through the object, and may include projection data or a sinogram. The data that has undergone back-projection is obtained by performing back-projection on the raw data by using information about an angle at which X-rays are emitted. The tomographic image is obtained by using image reconstruction techniques including back-projection of the raw data.

The storage 140 is a storage medium for storing control-related data, image data, etc., and may include a volatile or non-volatile storage medium.

The input interface 160 receives control signals, data, etc., from a user. The display 170 may display information indicating an operational status of the tomographic imaging system 100, medical information, medical image data, etc.

The tomographic imaging system 100 includes the communication interface 180 and may be connected to external devices, such as a server, a medical apparatus, and a portable device (smartphone, tablet personal computer (PC), wearable device, etc.), via the communication interface 180.

The communication interface 180 may include one or more components that enable communication with an external device. For example, the communication interface 180 may include a short distance communication module, a wired communication module, and a wireless communication module.

According to embodiments, the tomographic imaging system 100 may or may not use contrast media during a CT scan, and may be implemented as a device connected to other equipment.

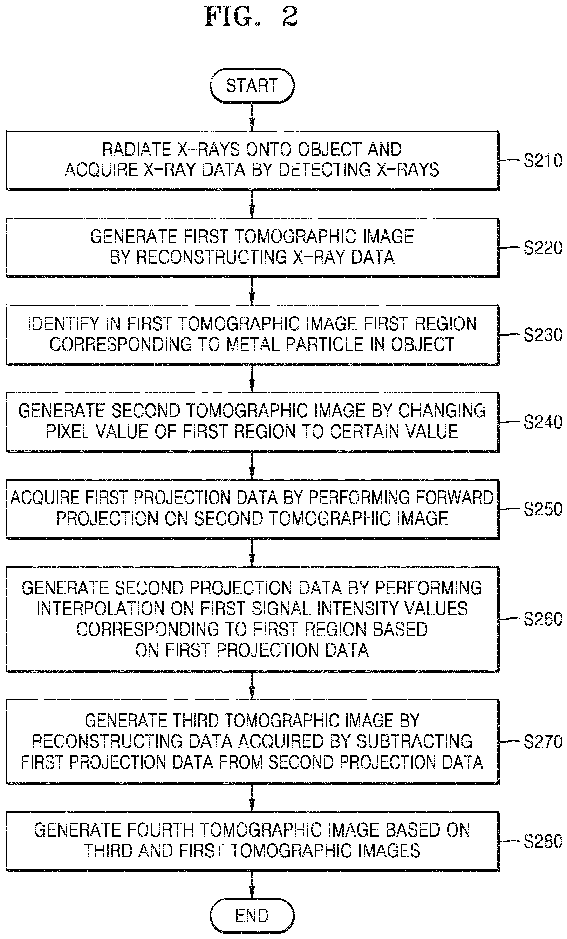

FIG. 2 is a flowchart of a method of generating a tomographic image, according to an embodiment.

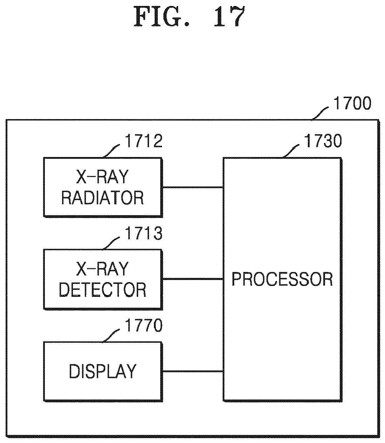

Referring to FIG. 2, a tomographic imaging apparatus, for example tomographic imaging apparatus 1700 of FIG. 17, may radiate X-rays onto an object and detect the radiated X-rays to thereby acquire X-ray data at operation S210.

The tomographic imaging apparatus 1700 may radiate X-rays toward the object via an X-ray radiator, for example X-ray radiator 1712 of FIG. 17. The tomographic imaging apparatus 1700 may radiate X-rays onto the object while rotating the X-ray radiator 1712 around a rotation axis.

The tomographic imaging apparatus 1700 may detect, via an X-ray detector, for example X-ray detector 1713 of FIG. 17, the X-rays radiated by the X-ray radiator 1712. The tomographic imaging apparatus 1700 may detect X-rays while rotating the X-ray detector 1713 around the rotation axis to thereby acquire a plurality of X-ray data. Each of the plurality of X-ray data may include an intensity value of an electrical signal obtained by converting X-rays detected by the X-ray detector 1713.

The tomographic imaging apparatus 1700 may generate a first tomographic image by reconstructing X-ray data at operation S220.

The tomographic imaging apparatus 1700 may generate the first tomographic image by applying an imaging reconstruction technique that includes an operation of performing back projection on X-ray data by using information about an angle at which X-rays are radiated by the X-ray radiator 1712. The first tomographic image may be composed of a plurality of pixels. Each of the pixels may include a value obtained by converting a signal intensity value in the X-ray data via the imaging reconstruction technique.

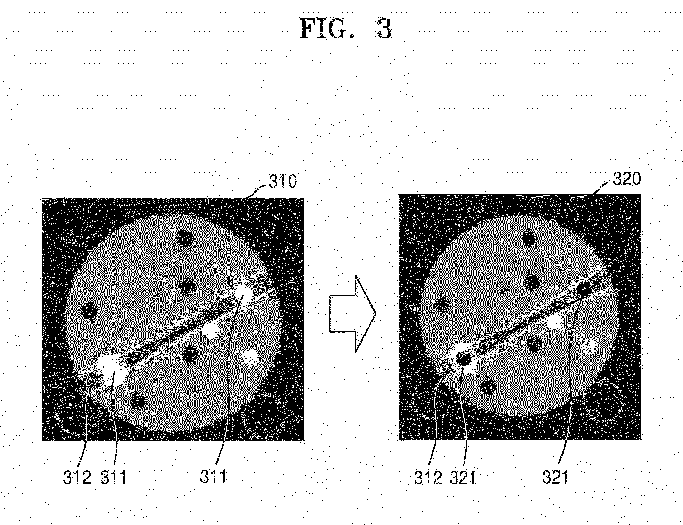

The tomographic imaging apparatus 1700 may identify, in the first tomographic image, a first region corresponding to a metal particle included in the object at operation S230. An example of operation S230 will be described in more detail below with reference to FIG. 3.

The tomographic imaging apparatus 1700 may generate a second tomographic image by changing a pixel value of the first region to a certain value, which may be for example a predetermined value, at operation S240. An example of operation S240 will be described in more detail below with reference to FIG. 3.

The tomographic imaging apparatus 1700 may acquire first projection data by performing forward projection on the second tomographic image at operation S250. The first projection data may include signal intensity values obtained by converting values of a plurality of pixels in the second tomographic image.

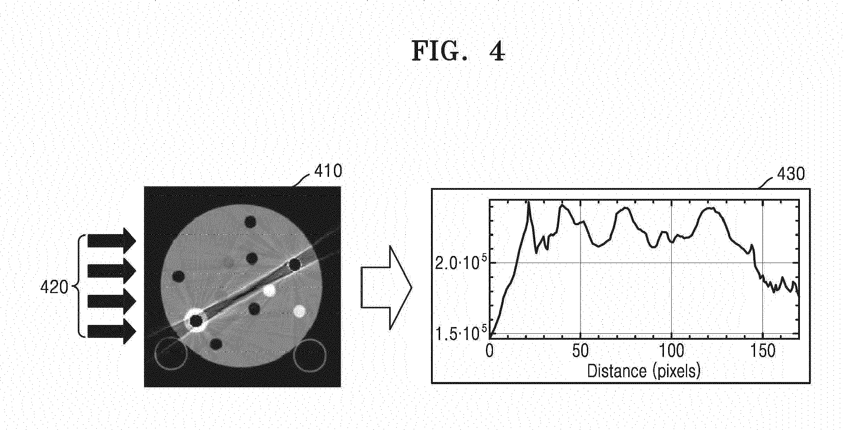

According to an embodiment, the tomographic imaging apparatus 1700 may perform forward projection on the second tomographic image by projecting a virtual parallel beam onto the second tomographic image, as described in more detail below with reference to FIG. 4.

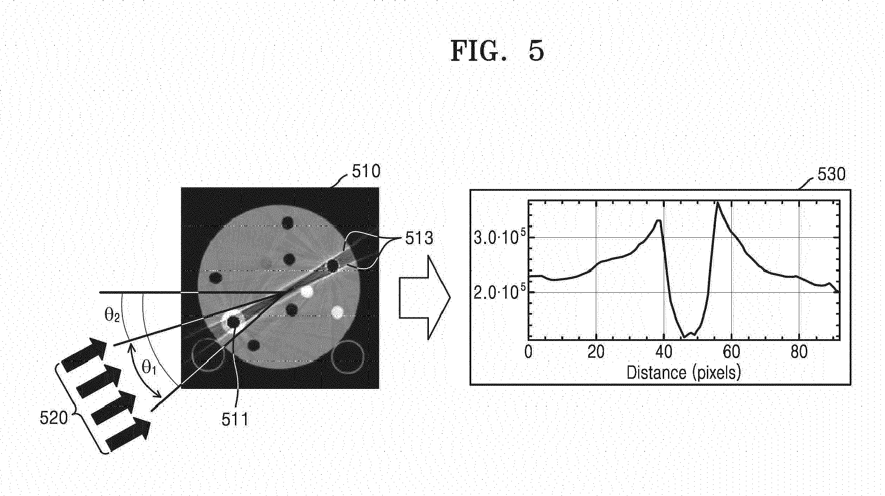

According to an embodiment, the tomographic imaging apparatus 1700 may set at least one projection angle at which forward projection is to be performed on the second tomographic image, based on a position of the first region corresponding to the metal particle in the second tomographic image. The tomographic imaging apparatus 1700 may perform forward projection on the second tomographic image at the set projection angle, as described in more detail below with reference to FIG. 5.

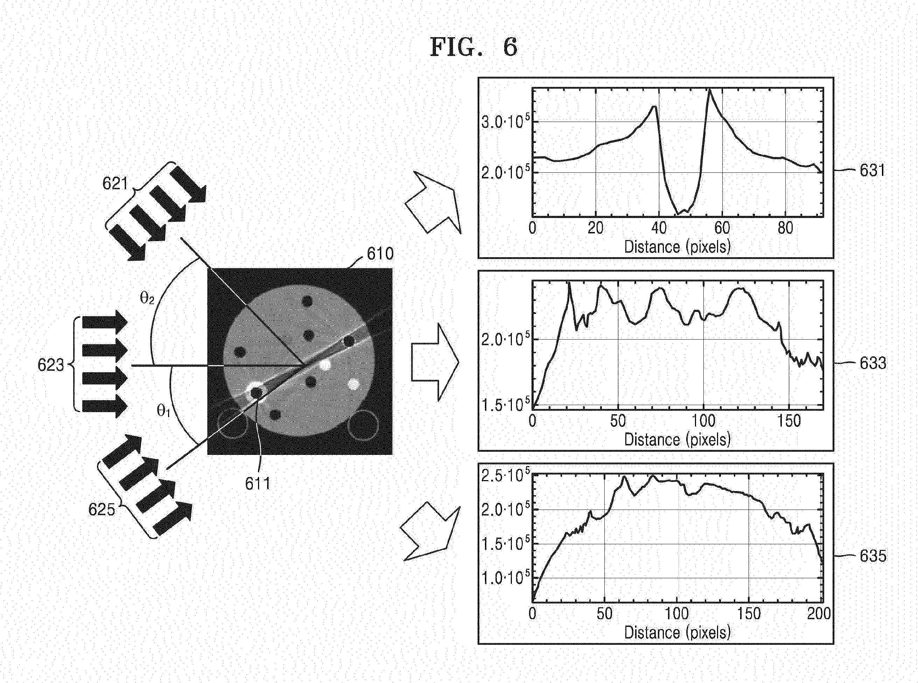

According to an embodiment, the tomographic imaging apparatus 1700 may set, based on the position of the first region in the second tomographic image, an interval of projection angles at which forward projection is to be performed on the second tomographic image. The tomographic imaging apparatus 1700 may perform forward projection on the second tomographic image at the set intervals, as described in more detail below with reference to FIG. 6.

The tomographic imaging apparatus 1700 may generate second projection data by performing interpolation on a signal intensity value corresponding to the first region based on the first projection data at operation S260.

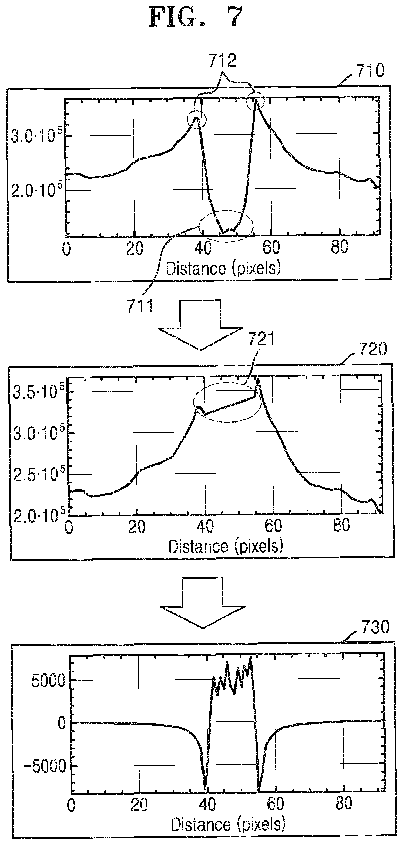

According to an embodiment, the tomographic imaging apparatus 1700 may interpolate first signal intensity values corresponding to the first region in the first projection data by using a signal intensity value in the first projection data, corresponding to a second region located near the first region, as described in more detail below with reference to FIG. 7.

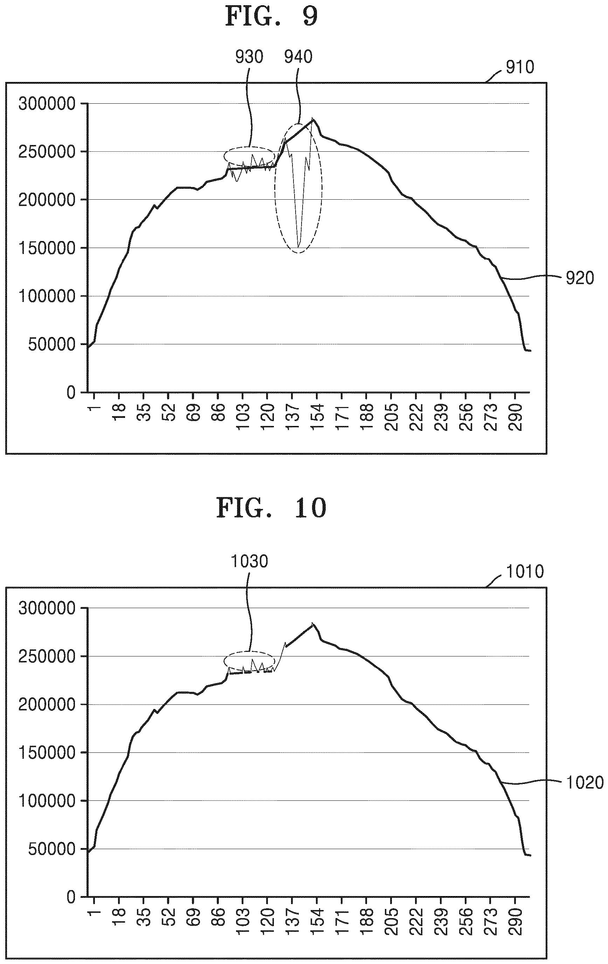

According to an embodiment, the tomographic imaging apparatus 1700 may change some of_second signal intensity values obtained by linearly interpolating first signal intensity values to their corresponding first signal intensity values, based on a result of comparing the first signal intensity values with the second signal intensity values. An example of the operation will be described in more detail below with reference to FIGS. 9 through 15.

The tomographic imaging apparatus 1700 may generate a third tomographic image by reconstructing data acquired by subtracting the first projection data from the second projection data at operation S270.

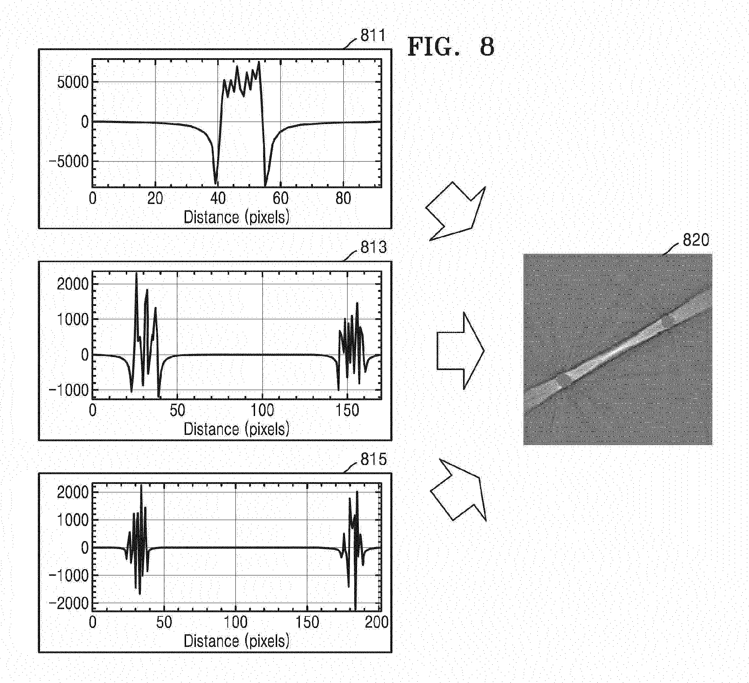

According to an embodiment, the tomographic imaging apparatus 1700 may generate third projection data by subtracting the first projection data from the second projection data. The tomographic imaging apparatus 1700 may generate a third tomographic image by performing back projection on the third projection data. The third tomographic image may include correction data for a metal artifact. An example of this operation will be described in more detail below with reference to FIGS. 7 and 8.

The tomographic imaging apparatus 1700 may generate a fourth tomographic image based on the third and first tomographic images at operation S280.

According to an embodiment, the tomographic imaging apparatus 1700 may generate the fourth tomographic image by combining the third tomographic image with the first tomographic image. The fourth tomographic image may be a tomographic image obtained by removing a metal artifact from the first tomographic image.

According to an embodiment, the tomographic imaging apparatus 1700 may additionally remove a metal artifact by applying, to the fourth tomographic image, image processing algorithms of the related art for removing metal artifacts, such as linear interpolation and smoothing.

The tomographic imaging apparatus 1700 may obtain a tomographic image from which a metal artifact is removed by performing the method of generating a tomographic image, according to the embodiment illustrated in FIG. 2. The user may accurately identify an internal structure of the object by using the tomographic image from which the metal artifact is removed.

According to the embodiment, it is possible to effectively remove a metal artifact caused by two or more metal particles in the object.

According to the embodiments, metal artifacts may be removed effectively without iterating forward and backward projections.

According to the embodiment, metal artifacts may be removed effectively even when a compact tomographic imaging apparatus having a low computing power (e.g., a dental tomographic imaging apparatus) is used.

FIG. 3 illustrates an example of changing a pixel value of a region corresponding to a metal particle in a tomographic image, according to an embodiment.

Referring to operation S230 of FIG.2, the tomographic imaging apparatus 1700 may identify a first region 311 corresponding to a metal particle in a first tomographic image 310.

According to an embodiment, the tomographic imaging apparatus 1700 may identify, as the first region 311 corresponding to the metal particle, a pixel having a value exceeding a threshold value from among pixels constituting the first tomographic image 310.

The tomographic imaging apparatus 1700 may identify the first region 311 corresponding to the metal particle by using various methods such as hard thresholding and soft thresholding.

According to an embodiment, the tomographic imaging apparatus 1700 may adjust a threshold value based on attribute data of a metal artifact. The attribute data of the metal artifact may include a value of a pixel corresponding to the metal artifact. In other words, the tomographic imaging apparatus 1700 may adjust a threshold value based on an intensity of the metal artifact. For example, when a white streak metal artifact is present in the first tomographic image 310 (i.e., when a third region corresponding to a metal artifact has a pixel value greater than that of a fourth region located near the third region), the tomographic imaging apparatus 1700 may identify the first region 311 based on a second threshold value that is greater than a first threshold value generally used to identify a metal region.

As another example, when a black shading metal artifact is present in the first tomographic image 310 (i.e., when the third region has a pixel value less than that of the fourth region), the tomographic imaging apparatus 1700 may identify the first region 311 based on a third threshold value that is less than the first threshold value.

According to an embodiment, the tomographic imaging apparatus 1700 may adjust a threshold value based on the appearance of an artifact that varies depending on a part of the object corresponding to the first tomographic image 310.

For example, when a portion corresponding to the first tomographic image 310 is a neck of the object (e.g., a human), a black shading metal artifact may occur in the first tomographic image 310. Thus, to remove the black shading metal artifact, the tomographic imaging apparatus 1700 may identify the first region 311 based on a third threshold value less than the first threshold value.

As another example, when a portion corresponding to the first tomographic image 310 is a spine of the object, a white streak metal artifact may occur in the first tomographic image 310 as a metal particle and a bone are adjacent to each other. Thus, to remove the white streak metal artifact, the tomographic imaging apparatus 1700 may identify the first region 311 based on the second threshold value greater than the first threshold value generally used to identify a metal region. As another example, when a black shading metal artifact and a white streak metal artifact appear together in the first tomographic image 310, the tomographic imaging apparatus 1700 may identify the first region 311 based on the third threshold value and perform operations S240 through S280 of FIG. 2. After performing operation S280, the tomographic imaging apparatus 1700 may identify the first region 311 based on the second threshold value and repeat operations S240 through S280.

The tomographic imaging apparatus 1700 may change a pixel value of the identified first region 311 to a certain value.

According to an embodiment, the tomographic imaging apparatus 1700 may refine the first region 311 identified based on a threshold value to correspond to a metal particle by applying techniques such as smoothing, erosion, and dilation to the first region 311. The tomographic imaging apparatus 1700 may then change a pixel value of the refined first region 311.

Referring to operation S240, the tomographic imaging apparatus 1700 may generate a second tomographic image 320 by changing a pixel value of the first region 311 to a certain value that is a pixel value of a first region 321.

According to an embodiment, the tomographic imaging apparatus 1700 may change the pixel value of the first region 311 to 0 or null. In other words, the tomographic imaging apparatus 1700 may generate the second tomographic image 320 by removing the pixel value of the first region 311 corresponding to the metal particle from the first tomographic image 310.

According to an embodiment, the tomographic imaging apparatus 1700 may set the certain value based on a pixel value of a second region 312 located near the first region 311.

For example, the tomographic imaging apparatus 1700 may set the certain value to be different from the pixel value of the second region 312 by a certain amount. In detail, when the second region 312 corresponds to a bone of the object, the tomographic imaging apparatus 1700 may set the certain value to 0 or Null. Furthermore, when the second region 312 corresponds to an organ of the object, the tomographic imaging apparatus 1700 may set the pixel value of the first region 311 to a certain value that is greater than or less than the pixel value of the second region 312.

FIG. 4 illustrates acquisition of projection data by performing forward projection on a tomographic image, according to an embodiment.

Referring to FIG. 4, the tomographic imaging apparatus 1700 may acquire first projection data 430 by performing forward projection of projecting a virtual parallel beam 420 onto a second tomographic image 410 at a certain angle.

The forward projection performed based on the virtual parallel beam 420 requires less computation than forward projection performed based on actually acquired X-ray data. Thus, according to the embodiment, metal artifacts may be removed effectively even when a compact tomographic imaging apparatus having a low computing power (e.g., a dental tomographic imaging apparatus) is used.

FIG. 5 illustrates setting of a projection angle at which forward projection is to be performed, according to an embodiment.

Referring to FIG. 5, the tomographic imaging apparatus 1700 may set at least one projection angle at which forward projection is to be performed on a second tomographic image 510, based on positions of a first region 511 corresponding to a metal particle in the second tomographic image 510.

For example, the tomographic imaging apparatus 1700 may set, based on the position of the first region 511 in the second tomographic image 510, a region θ₁-θ₂ where the forward projection is to be performed on the second tomographic image 510. The tomographic imaging apparatus 1700 may set a projection angle with respect to the set region θ₁-θ₂. The tomographic imaging apparatus 1700 may perform the forward projection on the second tomographic image 510 at the set projection angle. The tomographic imaging apparatus 1700 may acquire first projection data 530 by performing the forward projection on the second tomographic image 510.

According to an embodiment, the tomographic imaging apparatus 1700 may identify a region where a metal artifact 513 occurs based on the positions of the first region 511. When there are the first regions 511 in the second tomographic image 510, respectively corresponding to two metal particles, as shown in FIG. 5, the metal artifact 513 may occur between the metal particles.

When the metal artifact 513 appears in the form of lines along the first regions 511 as shown in FIG. 5, the tomographic imaging apparatus 1700 may perform forward projection on a region θ₁ where the metal artifact 513 is concentrated. In other words, the tomographic imaging apparatus 1700 may set a projection angle with respect to the region θ₁-θ₂ where the metal artifact 513 is concentrated. The tomographic imaging apparatus 1700 may perform forward projection by projecting a virtual parallel beam onto the second tomographic image 510 a plurality of times at certain intervals within the region θ₁-θ₂.

The forward projection performed only at a certain projection angle requires less computation than forward projection performed in all directions. Thus, according to the embodiment, metal artifacts may be removed effectively even when a compact tomographic imaging apparatus having a low computing power (e.g., a dental tomographic imaging apparatus) is used.

FIG. 6 illustrates setting of an interval of projection angles at which forward projection is to be performed, according to an embodiment.

Referring to FIG. 6, the tomographic imaging apparatus 1700 may set, based on a position of a first region 611 in the second tomographic image 610, an interval of projection angles at which beams 621, 623, and 625 are respectively projected onto a second tomographic image 610.

The tomographic imaging apparatus 1700 may acquire first projection data 631, 633, and 635 by respectively projecting the beams 621, 623, and 625 onto the second tomographic image 610 at the set intervals. Each of the first projection data 631, 633, and 635 may include a signal intensity value corresponding to the first region 611, which is obtained by converting a pixel value of the first region 611.

According to an embodiment, the tomographic imaging apparatus 1700 may set an interval between projection angles at which the beams 621, 623, and 625 are uniformly projected while rotating around the second tomographic image 610.

For example, the tomographic imaging apparatus 1700 may set an interval of projection angles to project a beam at intervals of 10° while rotating around the second tomographic image 610.

According to an embodiment, the tomographic imaging apparatus 1700 may set, based on the position of the first region 611 in the second tomographic image 610, different intervals of projection angles at which the beams 621, 623, and 625 are respectively projected onto the second tomographic image 610.

For example, the tomographic imaging apparatus 1700 may set, based on the position of the first region 611 in the second tomographic image 610, at least one region θ₁ and θ₂ where forward projection is to be performed on the second tomographic image 610. The tomographic imaging apparatus 1700 may set intervals of projection angles differently for each of the set regions θ₁ and θ₂.

In detail, when a metal artifact appears in the form of lines along metal particles as shown in FIG. 6, the tomographic imaging apparatus 1700 may set an interval of projection angles widely with respect to the region θ₂ where the metal artifact is dispersed while setting an interval of projection angles narrowly with respect to the region θ₁ where the metal artifact is concentrated.

FIG. 7 illustrates a subtraction image obtained by performing interpolation and then filtering on a signal intensity value in projection data, according to an embodiment.

Referring to FIG. 7, the tomographic imaging apparatus 1700 may generate second projection data 720 by performing interpolation on first signal intensity values 711 corresponding to a first region in first projection data 710. The tomographic imaging apparatus 1700 may generate third projection data 730 by subtracting the first projection data 710 from the second projection data 720. The third projection data 730 may include correction data for a metal artifact.

According to an embodiment, the tomographic imaging apparatus 1700 may interpolate the first signal intensity values 711 by using a signal intensity value corresponding to a second region located near the first region. The tomographic imaging apparatus 1700 may use a method of performing various interpolation techniques of the related art.

For example, the tomographic imaging apparatus 1700 may perform linear interpolation, bilinear interpolation, and spline interpolation, but is not limited thereto.

The third projection data 730 may be projection data to which a filter to be used for reconstructing a tomographic image is applied.

Because forward projection is performed on a second tomographic image generated by changing a pixel value of the first region to a certain value, the first signal intensity values 711 corresponding to the first region in the first projection data 710 has a large difference from a signal intensity value 712 corresponding to a second region. By performing linear interpolation on the first signal intensity values 711, the tomographic imaging apparatus 1700 may generate the second projection data 720 including second signal intensity values 721 similar to the signal intensity value 712 corresponding to the second region.

The tomographic imaging apparatus 1700 generates the third projection data 730 by subtracting the first projection data 710 from the second projection data 720, and thus, the third projection data 730 may include correction data for the metal artifact.

FIG. 8 illustrates generation of a tomographic image including correction data for a metal artifact, according to an embodiment.

Referring to FIG. 8, the tomographic imaging apparatus 1700 may generate a third tomographic image by reconstructing third projection data 811, 813, and 815. The tomographic imaging apparatus 1700 may reconstruct the third tomographic image 820 from the third projection data 811, 813, and 815 by using various tomographic image reconstruction techniques of the related art. The third tomographic image 820 may include correction data for a metal artifact.

FIG. 9 illustrates a comparison between projection data from which a signal intensity value corresponding to a metal particle is removed and projection data that has undergone interpolation, according to an embodiment.

In other words, FIG. 9 illustrates both first projection data 910, from which a signal intensity value corresponding to a metal particle is removed, and second projection data 920 acquired by linearly interpolating first signal intensity values.

Referring to FIG. 9, the first projection data 910 is different from the second projection data 920 in terms of a signal intensity value corresponding to a region that has undergone interpolation. The first projection data 910 may include at least one of third signal intensity values 930, which are values greater than second signal intensity values, the second signal intensity values being from among first signal intensity values, or fourth signal intensity values 940 which are less than the second signal intensity values.

The tomographic imaging apparatus 1700 may select at least one of the third signal intensity values 930 or the fourth signal intensity values 940 to remove a metal artifact.

The third signal intensity values 930 may be used to remove a white streak metal artifact. The white streak metal artifact means a metal artifact occurring when a pixel value of a third region corresponding to the metal artifact is greater than a pixel value of a fourth region located near the third region.

The fourth signal intensity values 940 may be used to remove a black shading metal artifact. The black shading metal artifact means a metal artifact occurring when a pixel value of the third region corresponding to the metal artifact is less than a pixel value of the fourth region located near the third region.

According to an embodiment, the tomographic imaging apparatus 1700 may select at least one of the third signal intensity values 930 or the fourth signal intensity values 940 based on a part of an object corresponding to the first tomographic image, for example first tomographic image 310 of FIG. 3.

For example, when a portion corresponding to the first tomographic image 310 is a neck of the object (e.g., a human), the tomographic imaging apparatus 1700 may select the fourth signal intensity values 940 based on frequent occurrence of a black shading metal artifact.

As another example, when a portion corresponding to the first tomographic image 310 is a spine of the object, a white streak metal artifact may occur in the first tomographic image 310 as a metal particle and a bone are adjacent to each other. Thus, the tomographic imaging apparatus 1700 may select the third signal intensity values 930.

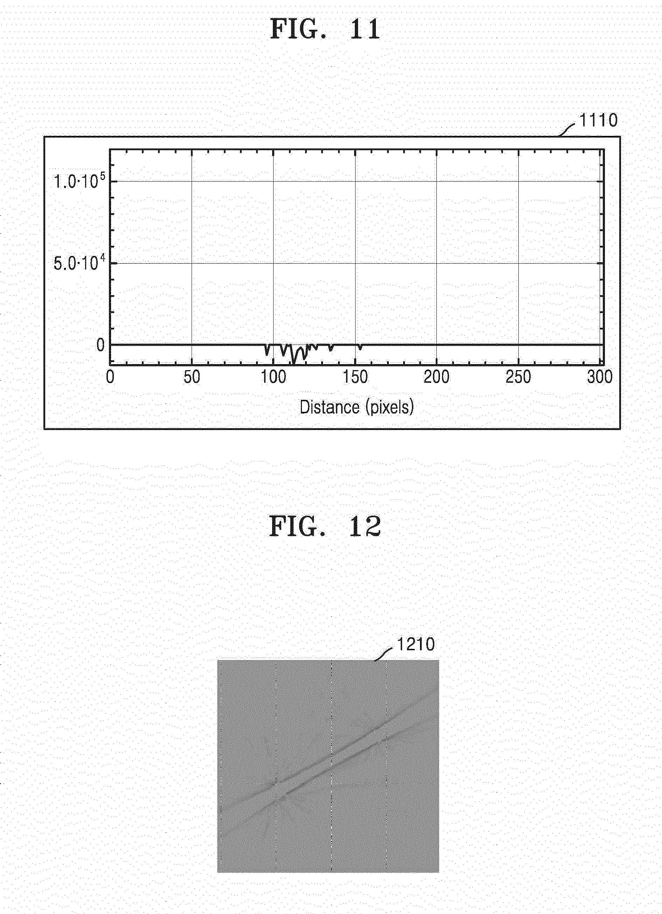

FIG. 10 illustrates projection data generated in order to remove a white streak metal artifact, according to an embodiment, and FIG. 11 illustrates projection data generated in order to remove a white streak metal artifact, according to an embodiment;

In detail, FIG. 10 illustrates second projection data 1010 acquired by calculating a difference between the first and second projection data (which may correspond to first projection data 910 and second projection data 920 of FIG. 9).

The tomographic imaging apparatus 1700 may compare first signal intensity values with second signal intensity values in order to remove a white streak metal artifact. The tomographic imaging apparatus 1700 may obtain fifth signal intensity values 1020 by calculating, based on a result of the comparing, a difference between second signal intensity values and third signal intensity values 1030 that are values greater than the second signal intensity values from among the first signal intensity values. The tomographic imaging apparatus 1700 may acquire the second projection data 1010 by updating the second projection data 920 to include the fifth signal intensity values 1020.

FIG. 11 illustrates third projection data 1110 acquired by the tomographic imaging apparatus 1700 subtracting the first projection data 910 from the second projection data 1010.

Referring to FIG. 11, the third projection data 1110 may include a certain pixel having a signal intensity value less than that of each neighboring pixel. The certain pixel may be a pixel corresponding to a region where a white streak metal artifact occurs, and a signal intensity value of the certain pixel may be correction data for removing the white streak metal artifact.

FIG. 12 illustrates a tomographic image including correction data for removing a white streak metal artifact, according to an embodiment.

The tomographic imaging apparatus 1700 may reconstruct a third tomographic image 1210 from the third projection data, for example third projection data 1110 of FIG. 11. The third tomographic image 1210 may include correction data for removing a white streak metal artifact.

Referring to FIG. 12, the third tomographic image 1210 may include a first pixel with a value less than that of each neighboring pixel. The first pixel may correspond to a region where the white streak metal artifact occurs.

The tomographic imaging apparatus 1700 may remove a white streak metal artifact by adding together attribute data of the white streak metal artifact and a pixel value complementary to the white streak metal artifact. Thus, the value of the first pixel may correspond to the correction data for removing a white streak metal artifact.

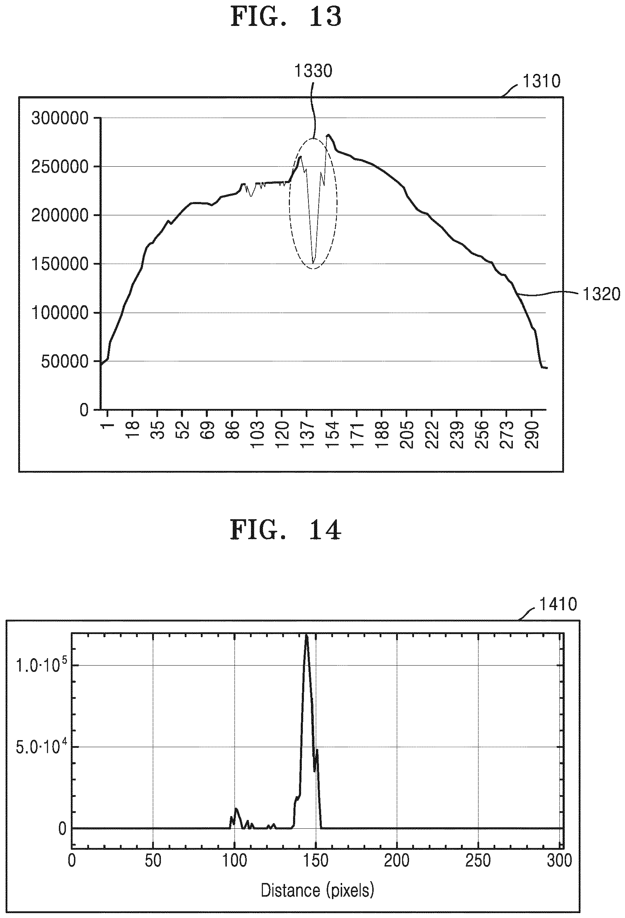

FIG. 13 illustrates projection data generated for removing a black shading metal artifact, according to an embodiment, and FIG. 14 illustrates projection data generated for removing a black shading metal artifact, according to an embodiment.

In detail, FIG. 13 illustrates second projection data acquired by calculating a difference between the first and second projection data 910 and 920.

The tomographic imaging apparatus 1700 may compare first signal intensity values with second signal intensity values in order to remove a black shading metal artifact. The tomographic imaging apparatus 1700 may obtain fifth signal intensity values 1320 by calculating, based on a result of the comparing, a difference between second signal intensity values and fourth signal intensity values 1330 that are values less than the second signal intensity values from among the first signal intensity values. The tomographic imaging apparatus 1700 may acquire the second projection data 1310 by updating the second projection data 920 to include the fifth signal intensity values 1320.

FIG. 14 illustrates third projection data 1410 that the tomographic imaging apparatus 1700 acquires by subtracting the first projection data 910 from the second projection data 1310.

Referring to FIG. 14, the third projection data 1410 may include a certain pixel with a signal intensity value greater than that of each neighboring pixel. The certain pixel may be a pixel corresponding to a region where a black shading metal artifact occurs, and a signal intensity value of the certain pixel may be correction data for removing the black shading metal artifact.

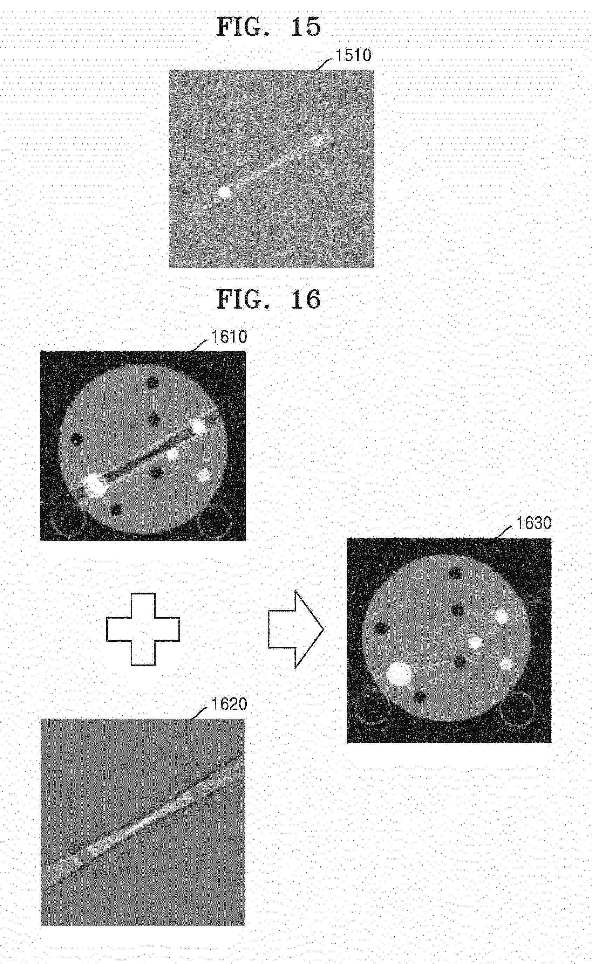

FIG. 15 illustrates a tomographic image including a correction value for removing a black shading metal artifact, according to an embodiment.

The tomographic imaging apparatus 1700 may reconstruct a third tomographic image 1510 from the third projection data, for example third projection data 1410 of FIG. 14. The third tomographic image 1510 may include correction data for removing a black shading metal artifact.

Referring to FIG. 15, the third tomographic image 1510 may include a second pixel with a value greater than that of each neighboring pixel. The second pixel may correspond to a region where the black shading metal artifact occurs.

The tomographic imaging apparatus 1700 may remove a black streak metal artifact by adding together attribute data of the black shading metal artifact and a complementary pixel value. Thus, the value of the second pixel may correspond to the correction data for removing a black shading metal artifact.

FIG. 16 illustrates a tomographic image from which a metal artifact is removed, according to an embodiment.

Referring to FIG. 16, the tomographic imaging apparatus 1700 may generate a fourth tomographic image 1630 from which a metal artifact is removed based on first tomographic image 1610 and third tomographic image 1620.

For example, the tomographic imaging apparatus 1700 may generate the fourth tomographic image 1630 from which a metal artifact is removed by adding together the first tomographic image 1610 and the third tomographic image 1620 that includes a pixel value complementary to the metal artifact in the first tomographic image 1610.

Referring to FIGS. 10 through 12, the tomographic imaging apparatus 1700 may generate a fourth tomographic image from which only a white streak metal artifact is selectively removed.

Referring to FIGS. 13 through 15, the tomographic imaging apparatus 1700 may generate a fourth tomographic image from which only a black shading metal artifact is selectively removed.

The tomographic imaging apparatus 1700 may simultaneously or sequentially perform a method of removing a black shading metal artifact and a method of removing a white streak metal artifact.

The tomographic imaging apparatus 1700 may additionally remove a metal artifact by applying, to the fourth tomographic image 1630, image processing algorithms of the related art for removing metal artifacts, such as linear interpolation and smoothing.

FIG. 17 is a block diagram of a structure of the tomographic imaging apparatus 1700 according to an embodiment.

Referring to FIG. 17, the tomographic imaging apparatus 1700 may be an apparatus for performing at least one of the functions of the tomographic imaging system 100 of FIG. 1. Thus, the tomographic imaging apparatus 1700 may include components corresponding to at least one component included in the tomographic imaging system 100 of FIG. 1. For example, the tomographic imaging apparatus 1700 may include the X-ray radiator 1712, the X-ray detector 1713, a display 1770, and a processor 1730.

The X-ray radiator 1712 may include at least one of the components necessary for radiating X-rays onto an object. For example, the X-ray radiator 1712 may include at least one of the gantry 110, the rotating frame 111, the X-ray generator 112, or the rotation driver 114 described with reference to FIG. 1.

The X-ray detector 1713 may include at least one of the components necessary for detecting the X-rays radiated by the X-ray radiator 1712 onto the object. For example, the X-ray detector 1713 may include at least one of the X-ray detector 113, the readout device 115, the DAS 115-1, or the data transmitter 115-2 described with reference to FIG. 1.

The processor 1730 may control all operations of the tomographic imaging apparatus 1700 and a signal flow between the components inside the tomographic imaging apparatus 1700. The processor 1730 may include a memory for storing programs or data for performing functions of the tomographic imaging apparatus 1700. For example, the processor 1730 may include at least one of the controller 130, the storage 140, or the image processor 150.

The processor 1730 may include at least one of a general-purpose processor (e. g., a central processing unit (CPU) or a special-purpose processor manufactured to capture a tomographic image and perform image processing. The processor 1730 may include a plurality of processors 1730 operating as a single processor.

The processor 1730 may execute instructions for performing the method of generating a tomographic image according to at least one of the embodiments.

For example, the processor 1730 may reconstruct a first tomographic image from X-ray data.

The processor 1730 may identify, in the first tomographic image, a first region corresponding to a metal particle included in the object.

The processor 1730 may generate a second tomographic image by changing a pixel value of the first region to a certain value. The processor 1730 may set the certain value based on attribute data of a metal artifact in the first tomographic image. Alternatively, the processor 1730 may set the certain value based on a pixel value of a second region located near the first region. Alternatively, the processor 1730 may set the certain value based on a part of the object corresponding to the first tomographic image.

The processor 1730 may acquire first projection data by performing forward projection on the second tomographic image. The processor 1730 may perform forward projection on the second tomographic image by projecting a virtual parallel beam onto the second tomographic image. The processor 1730 may set, based on a position of a metal particle, at least one projection angle at which forward projection is to be performed on the second tomographic image and acquire the first projection data by performing the forward projection on the second tomographic image at the set projection angle. The processor 1730 may set, based on a position of a metal particle, an interval of projection angles at which forward projection is to be performed on the second tomographic image and acquire the first projection data by performing the forward projection on the second tomographic image at the set intervals.

The processor 1730 may generate second projection data by performing interpolation on first signal intensity values corresponding to the first region based on the first projection data.

The processor 1730 may compare first signal intensity values in the first projection data with second signal intensity values obtained by performing linear interpolation on the first signal intensity values.

The processor 1730 may select, based on the attribute data of the metal artifact in the first tomographic image, third signal intensity values that are values greater than second signal intensity values, the second signal intensity values being from among the first signal intensity values or fourth signal intensity values that are values less than the second signal intensity values.

The processor 1730 may obtain fifth signal intensity values by calculating a difference between the selected third or fourth signal intensity values and the second signal intensity values and generate the second projection data based on the obtained fifth signal intensity values.

The processor 1730 may select the third signal intensity values based on a comparison result that a pixel value of a third region is greater than that of a fourth region located near the third region, and the fourth signal intensity values based on a comparison result that the pixel value of the third region is less than that of the fourth region.

The processor 1730 may generate a third tomographic image including correction data for the metal artifact caused by the metal particle by reconstructing data acquired by subtracting the first projection data from the second projection data.

The processor 1730 may generate a fourth tomographic image by adding the third tomographic image to the first tomographic image.

The processor 1730 may additionally remove a metal artifact by applying, to the fourth tomographic image, image processing algorithms of the related art for removing metal artifacts, such as linear interpolation and smoothing.

The processor 1730 may control operations of the tomographic imaging apparatus 1700 based on a control signal received via the input interface 160 of FIG. 1 or a control signal received from an external device.

The embodiments may be implemented as a software program including instructions stored in a computer-readable storage medium.

A computer may refer to a device configured to retrieve an instruction stored in the computer-readable storage medium and to operate, in response to the retrieved instruction, and may include an tomographic imaging apparatus according to embodiments.

The computer-readable storage medium may be provided in the form of a non-transitory storage medium. In this regard, the term "non-transitory" means that the storage medium does not include a signal and is tangible, and the term does not distinguish between data that is semi-permanently stored and data that is temporarily stored in the storage medium.

In a system consisting of a server and a terminal (e.g., the tomographic imaging apparatus), the computer program product may include a storage medium of the server or a storage medium of the terminal. Alternatively, in a case where a third device (e.g., a smartphone) that communicates with the server or the terminal is present, the computer program product may include a storage medium of the third device. Alternatively, the computer program product may include a software program that is transmitted from the server to the terminal or the third device or that is transmitted from the third device to the terminal.

In this case, one of the server, the terminal, and the third device may execute the computer program product, thereby performing the method according to embodiments. Alternatively, at least two of the server, the terminal, and the third device may execute the computer program product, thereby performing the method according to embodiments in a distributed manner.

For example, the server (e.g., a cloud server, an artificial intelligence (AI) server, or the like) may execute the computer program product stored in the server, and may control the terminal to perform the method according to embodiments, the terminal communicating with the server.

As another example, the third device may execute the computer program product, and may control the terminal to perform the method according to embodiments, the terminal communicating with the third device. In more detail, the third device may remotely control the tomographic imaging apparatus to emit X-ray to an object, and to generate an image of an inner part of the object, based on detected radiation which passes the object and is detected in an X-ray detector.

As another example, the third device may execute the computer program product, and may directly perform the method according to embodiments, based on at least one value input from an auxiliary device (e.g., a gantry of tomographic imaging system). In more detail, the auxiliary device may emit X-ray to an object and may obtain information of radiation which passes the object and is detected in an X-ray detector. The third device may receive an input of signal information about the detected radiation from the auxiliary device, and may generate an image of an inner part of the object, based on the input radiation information.

In a case where the third device executes the computer program product, the third device may download the computer program product from the server, and may execute the downloaded computer program product. Alternatively, the third device may execute the computer program product that is pre-loaded therein, and may perform the method according to the embodiments.

While embodiments of the present disclosure have been particularly shown and described with reference to the accompanying drawings, it will be understood by those of ordinary skill in the art that various changes in form and details may be made therein without departing from the spirit and scope as defined by the appended claims. The disclosed embodiments should be considered in descriptive sense only and not for purposes of limitation.

## Claims

1. A method of generating a tomographic image, the method being performed by a tomographic imaging apparatus and comprising:
radiating X-rays onto an object and acquiring X-ray data by detecting the X-rays has passed through the object;
generating a first tomographic image by reconstructing the X-ray data;
identifying, in the first tomographic image, a first region corresponding to a metal particle included in the object;
generating a second tomographic image by changing a pixel value of the first region to a predetermined value;
acquiring first projection data by performing forward projection on the second tomographic image;
generating second projection data by performing interpolation on first signal intensity values corresponding to the first region, based on the first projection data;
generating reconstruction data by subtracting the first projection data from the second projection data;
generating a third tomographic image based on the reconstruction data, wherein the third tomographic image includes correction data corresponding to a metal artifact caused by the metal particle; and
generating a fourth tomographic image based on the third tomographic image and the first tomographic image.

2. The method of claim 1, wherein the generating of the second tomographic image comprises setting the predetermined value based on attribute data of the metal artifact in the first tomographic image.

3. The method of claim 1, wherein the generating of the second tomographic image comprises setting the predetermined value based on a pixel value of a second region located near the first region.

4. The method of claim 1, wherein the generating of the second tomographic image comprises setting the predetermined value based on a part of the object corresponding to the first tomographic image.

5. The method of claim 1, wherein the acquiring of the first projection data comprises performing the forward projection on the second tomographic image by projecting a virtual parallel beam onto the second tomographic image.

6. The method of claim 1, wherein the acquiring of the first projection data comprises:
setting, based on a position of the metal particle, at least one projection angle of the forward projection; and
acquiring the first projection data by performing the forward projection on the second tomographic image at the at least one projection angle.

7. The method of claim 1, wherein the acquiring of the first projection data comprises:
setting, based on a position of the metal particle, an interval of projection angles of the forward projection; and
acquiring the first projection data by performing the forward projection on the second tomographic image at the interval.

8. The method of claim 1, wherein the generating of the second projection data comprises:
comparing the first signal intensity values in the first projection data with second signal intensity values obtained by performing linear interpolation on the first signal intensity values;
selecting, based on attribute data of the metal artifact in the first tomographic image, one from among third signal intensity values greater than the second signal intensity values from among the first signal intensity values, or fourth signal intensity values less than the second signal intensity values from among the first signal intensity values;
obtaining fifth signal intensity values by calculating a difference between the second signal intensity values and the selected one from among the third signal intensity values or the fourth signal intensity values; and
generating the second projection data based on the obtained fifth signal intensity value.

9. The method of claim 8, wherein the attribute data of the metal artifact comprises a pixel value of a third region corresponding to the metal artifact, and
wherein the selecting of the one from among the third signal intensity values or the fourth signal intensity values comprises:
selecting the third signal intensity values based on a comparison result indicating that the pixel value of the third region is greater than a pixel value of a fourth region located near the third region; and
selecting the fourth signal intensity values based on a comparison result indicating that the pixel value of the third region is less than the pixel value of the fourth region.

10. A computer program product comprising a computer-readable storage medium having recorded thereon instructions for executing the method of claim 1 on a computer.

11. A tomographic imaging apparatus comprising:
an X-ray radiator configured to radiate X-rays onto an object;
an X-ray detector configured to acquire X-ray data by detecting the X-rays has passed through the object; and
at least one processor configured to:
generate a first tomographic image by reconstructing the X-ray data; identify, in the first tomographic image, a first region corresponding to a metal particle included in the object;
generate a second tomographic image by changing a pixel value of the first region to a predetermined value;
acquire first projection data by performing forward projection on the second tomographic image;
generate second projection data by performing interpolation on first signal intensity values corresponding to the first region based on the first projection data;
generate reconstruction data by subtracting the first projection data from the second projection data;
generate a third tomographic image based on the reconstruction data, wherein the third tomographic image includes correction data corresponding to a metal artifact caused by the metal particle; and
generate a fourth tomographic image based on the third tomographic image and the first tomographic image.

12. The tomographic imaging apparatus of claim 11, wherein the at least one processor is further configured to set the predetermined value based on attribute data of the metal artifact in the first tomographic image.

13. The tomographic imaging apparatus of claim 11, wherein the at least one processor is further configured to set the predetermined value based on a pixel value of a second region located near the first region.

14. The tomographic imaging apparatus of claim 11, wherein the at least one processor is further configured to set the predetermined value based on a part of the object corresponding to the first tomographic image.

15. The tomographic imaging apparatus of claim 11, wherein the at least one processor is further configured to perform the forward projection on the second tomographic image by projecting a virtual parallel beam onto the second tomographic image.
